# EUROPEAN PATENT SPECIFICATION

(11) **EP 1 030 712 B1**
(45) Date of publication and mention of the grant of the patent: **07.01.2009**
(21) Application number: 98948157.7
(22) Date of filing: 10.09.1998
(51) Int. Cl.: A61N 1/00, A61N 2/00, A61N 2/06

(54) **APPARATUS FOR ALTERING THE CHARGE DISTRIBUTION UPON LIVING MEMBRANES WITH FUNCTIONAL STABILIZATION OF THE MEMBRANE PHYSICAL ELECTRICAL INTEGRITY**
GERÄT ZUR VERÄNDERUNG DER LADUNGSVERTEILUNG AUF LEBENDEN MEMBRANEN MIT FUNKTIONELLER STABILISIERUNG DER PHYSIKALISCHEN INTEGRITÄT DER MEMBRAN
APPAREIL PERMETTANT DE MODIFIER LA REPARTITION DE CHARGE SUR DES MEMBRANES VIVANTES AVEC UNE STABILISATION FONCTIONNELLE DE L'INTEGRITE ELECTROPHYSIQUE DE LA MEMBRANE

(30) Priority: 10.09.1997 US 926633
(43) Date of publication of application: 30.08.2000
(73) Proprietor: Holcomb, Robert R., Nashville, TN 37212 (US)
(72) Inventor: Holcomb, Robert R., Nashville, TN 37212 (US)
(74) Representative: Harrison Goddard Foote
(86) International application number: PCT/US1998/018967
(87) International publication number: WO 1999/012605

(56) References cited:
- EP-A- 0 428 474
- WO-A-97/00639
- WO-A-97/04830
- US-A- 5 312 321
- No search

## Description

### BACKGROUND OF THE INVENTION

### 1. Field of the Invention

The present invention relates to magnetic devices for therapeutic application to the human body, and more particularly to a static field, quadripolar magnetic treatment device with flux return means and a focusing means to increase the intensity, focus and gradient of the field for placement against or in proximity to the human or other animal body.

Such magnetic treatment devices may be used for the treatment of various animal diseases, complications and disorders such as, but not limited to, a) acute and chronic pain. b) cardiac dysfunction, c) seizure disorders, d) pain and edema sustained in minor burns, insect bites and bee stings) potentiation of pharmaceuticals and focusing and for concentrating the drug to the active site, f) protection of transplant organs, g) treatment of movement disorders. h) control of edema and pain as well as speed healing following surgical procedures. i) control of pain and sludging of sickled cells in sickle cell disease, j) foot pain and discomfort, k) Magna Scan device in the treatment of pain and other dysfunctions, I) potentiation of epidural anesthesia and epidural analgesia, m) Protection from cell injury and death following cell insults such as contusion, hypoxic stroke and infection, n) control of nausea and vomiting associated with pregnancy, motion, and chemotherapy, o) prevention of fertilization of ovum by sperm, p) cumulative trauma disorder in the workplace, q) a magnetic placebo which has no biological activity yet is magnetic and has all characteristics of the authentic device except the alternating poles and a significant field gradient.

### 2. General Background of the Invention

Magnetic fields have been applied to the human body for various therapeutic purposes for many centuries. For example, magnetic medical treatment devices for application against selected portions of the human body are disclosed in United States Patent No. 3,921,620; method and apparatus for suppressing neuron action potential firings 5,312,321; magnetic plasters for improving circulation are disclosed in United States Patent No. 4,489, 711; magnetic fields for stimulation of bone growth are disclosed in U.S. Patent No. 4,105,017; and magnetic stimulation of nerve cells has been accomplished with devices such as the Cadwell Magneto-Electric Stimulator (MES-10) manufactured by Cadwell Laboratories, Inc. Of Kennewick, Washington.

It is known from WO 97/00639 10 provide an electromagnetic therapeutic treatment device. Various disease states, tissue and organ malfunction may be the result of loss of membrane stability and normal permeability. These membranes may be cellular of intracellular, but in any case represent malfunction of excitable tissue. This malfunction of excitable tissue may be due to alteration of ion channel function. These various disease and states of malfunction may also be related to alteration of receptor sites or agonist sites of enzymes and/or other such dynamic systems within living organisms and more particularly the human animal. A great variety of symptoms and malfunctions may occur, such as, but not limited to, the above listed disease and/or disorder states.

Unfortunately, many types of ailments, including chronic pain, poor localized blood flow, cerebral edema and certain seizures and injuries cannot be successfully treated with conventional drug, physical therapy or surgical therapies. Because such ailments are often untreatable with conventional therapies, there is a need for alternative therapies that relieve these previously untreatable or poorly treatable conditions.

Accordingly, it is an object of this invention to provide a device that alters the stability of excitable membranes and other charged structures and systems in order to treat ailments of animals.

Another object of the invention is to provide a static magnetic device for production of a magnetic field for treatment of such disorders, such a device being powered by a particular static magnetic field and having, inter alia, an alternating polarity, quadripolar array which generates a 3 dimensional, steep field gradient (greater than 0.25mt/mm) with a homogenous field.

It is a further object of this invention to provide an alternating, quadripolar array in which each of the poles is in the shape of two cones joined at the derectrix of the cone with the vertex of the two cones lying in a perpendicular axis of a circular directrix.

An additional object of this invention is to provide a device with four magnetic poles of alternating polarity in which the shape of the poles is in the form of two cones with the directrix with the vertex of the two cones lying in a perpendicular axis of a circular directrix of the cones such that the vertex of the cone shaped magnetic bodies may all be tilted toward (or away from) the midline (on the side facing the animal body) such that the peak or vertex migration toward the midline will vary the field gradient.

It is further object of this invention to provide a device which contains a flux return ring on the back surface away from the body surface which is designed to return the magnetic flux thereby altering the strength and gradient without materially altering the center charge symmetry and homogeneity of the 3 dimensional steep gradient field which is in contact with the animal body.

Another object of the invention is to provide a flux focusing ring surrounding the 4 magnetic poles on the outer perimeter stationed substantially midway between the top and bottom of the pole. Attachment means is provided to hold the focusing ring to the outer perimeter of the poles throughout the total support means.

It is a further object of the invention to provide a static magnetic pole of like polarity on the outer surface of the flux focusing ring adjacent to each of the 4 poles of the invention (focusing magnet) such that the end or top of the focusing magnet is oriented to the geometric side of the pole such that the axis of the two magnets form a 45 to 90 degree angle. The focusing magnet comprises a static magnetic pole. The angle of the focusing magnet is such that the axis forms a 45 to 90 degree angle on the body surface side and a 90 to 135 degree to the surface away from the body.

It is a further object of the invention to control the focusing of each magnet with a smaller and weaker magnet than the primary pole such that the focusing ring containing magnets can focus and balance the symmetry of the therapeutic field.

There may be added a plastic material to a permanent magnet amalgum along with a proprietary silica colloid and curing the amalgum under the influence of a strong, center charged homogenous magnetic field.

It is a further feature to reveal the importance and magnitude of the gradient in the "Z" axis and to present data on the importance and magnitude of the 3 dimension flux field gradients.

It is a further object of the invention to present an application of the quadripolar array in a microscopic embodiment which is housed in a catheter designed to be placed in the epidural space and other body cavities such that the magnetic quadripolar flux generators are arranged in a helix such that migration or rotation of the device will not alter the efficacy of the therapeutic device.

Another object of the invention is to present a device in which the desired biological effects are directly related to the magnitude of the gradient in the x, y and z axis and therefore the magnitude of the vector or summation gradient.

Various embodiments of the use of the core static magnetic field produced in the instant invention include the following, but are not limited to the following.

### A. Pain and Swelling and Wound Heating

### 1. Acute Pain and Edema

It is a further object of the invention to provide a device that alters nerve cell behavior in a manner that reduces painful sensations.

Another object of the invention is to provide an apparatus for applying a symmetric quadripolar, three dimensional magnetic flux field which is focused and balanced to the human body to stabilize excitable membranes and thereby reduce pain and edema associated with acute injury, inflammation or surgical procedure and to decrease wound healing time.

Another object of the invention is to provide a specialized magnetic flux field to control and reverse the swelling associated with acute injury, inflammation and surgery.

It is a further object of this invention to provide an alternating, quadripolar array in which each of the poles is in the shape of two cones joined at the directrix of the cone with the vertex of the two cones lying in a perpendicular axis of a circular directrix.

An additional object of this invention is to provide a device with four magnetic poles of alternating polarity in which the shape of the poles is in the form of two cones joined at the directrix with the vertex of the two cones lying in a perpendicular axis of a circular directrix of the cones such that the vertex of the cone shaped magnetic bodies may all be tilted toward the midline (on the side facing the animal body) such that the peak or vertex migration toward the midline will alter the field gradient.

It is further object of this invention to provide a device which contains a flux return ring on the back surface away from the body surface which is designed to return the magnetic flux thereby increasing the strength and gradient without altering the center charge symmetry and homogeneity of the 3 dimensional steep gradient field.

Another object of the invention is to provide a flux focusing ring surrounding the 4 static magnetic poles on the outer perimeter stationed essentially midway between the top and bottom of the pole. Attachment means is provided to hold the focusing ring to the outer perimeter of the poles through the total support means.

It is a further object of the invention to provide a static magnetic pole of like polarity on the outer surface of each of the 4 poles of the invention such that the top of the core is oriented to the geometric side of the pole such that the axis of the two magnets form a 45 to 90 degree angle. The focusing magnet comprises a static magnetic pole.

It is a further object of the invention to control the focusing of each magnet with a smaller and weaker magnet than the primary pole such that the focusing ring containing magnets can focus and balance the symmetry of the therapeutic field.

There is disclosed a method for applying a therapeutic magnetic device to the human body to relieve pain, improve blood flow, decrease healing time, and reduce swelling associated with injury, surgery or acute inflammation.

### 2. Chronic Pain

It is a further object of the invention to provide a device that alters nerve cell behavior in cases of chronic pain to block the spontaneous pacemaker firing of the chronically malfunctioning pain fiber (mostly A-fibers and C-libers).

Another object of the invention is to provide an apparatus for applying a symmetric, quadripolar, three dimensional magnetic flux field which is focused and balanced to the human body to stabilize excitable membranes and thereby reduce pain associated with the spontaneous pacemaker firing of chronically malfunctioning polymodal nociceptors (afferent C-fibers and A8 libers).

Another object of the invention is to provide a specialized magnetic flux field to control and speed healing of chronic non-healing wounds.

It is a further object of this invention to provide an alternating, quadripolar array in which each of the poles is in the shape of two cones joined at the directrix of the cone with the vertex of the two cones lying in a perpendicular axis of a circular directrix.

An additional object of this invention is to proved a device with four magnetic poles of alternating polarity in which the shape of the poles is in the form of two cones joined at the directrix with the vertex of the two cones lying in a perpendicular axis of a circular directrix of the cones such that the vertex of the cone shaped magnetic bodies may all be tilted toward the midline (on the side facing the animal body) such that the peak or vertex migration toward the midline will alter the field gradient.

It is further object of this invention to provide a device which contains a flux return ring on the back surface away from the body surface which is designed to return the magnetic flux thereby increasing the strength and gradient without altering the center charge symmetry and homogeneity of the 3 dimensional steep gradient field,

Another object of the invention is to provide a flux focusing ring surrounding the 4 static magnetic poles on the outer perimeter stationed essentially midway between the top and bottom of the pole. Attachment means is provided to hold the focusing ring to the outer perimeter of the poles throughout the total support means.

It is a further object of the invention to provide a static magnetic pole of like polarity on the outer surface of each of the 4 poles of the invention such that the top of the core is oriented to the geometric side of the pole such that the axis of the two magnets form a 45 to 90 degree angle. The focusing magnet comprises a static magnetic pole.

It is a further object of the invention to control the focusing of each magnet with a smaller and weaker magnet than the primary pole such that the focusing ring containing magnets can focus and balance the symmetry of the therapeutic field.

There is disclosed a method for applying a therapeutic magnetic device to the human body to relieve chronic pain. improve blood flow and decrease healing time.

### L. Potentiation of Epidural Anesthesia and Epidural Analgesia.

Embodiments of the present invention relates to submicroscopic, quadripolar, circular, center charged, energy balanced magnetic devised in a four (4) magnet array of alternating polarity in which the magnetic poles are separated only by a distance which will allow a magnetic sphere of influence on all adjacent poles to suppress the firing of action potentials of mammalian sensory neurons. These submicroscopic magnetic arrays are placed in an epidural catheter. They are placed in a helical array with the distance between magnetic clusters being 1 cm (a distance which breaks the sphere of influence, one cluster field to another). The cluster are rotated about 43° in succession one cluster to the next. There is a difference in electrical potential across a cell membrane of sensory neurons. When a neuron receives an impulse transmitted from another nerve cell, the electrical potential difference across the membrane of the cell is dramatically reduced and generally reverses. This reduction and reversal of potential is referred to as the firing of the neurons action potential. If such action potential firings are suppressed, the transmissions of nerve impulses are also suppressed

Pain sensations in the human body can be a result of improper nerve function, as when such pain is caused by inordinately excitable nerve cells or by nerve cells having cell wall membranes that leak ions. Pain sensations may also be caused by damaged nerve cells, as for example nerve cells suffering from post-operative scarring or physically impinged nerve cells commonly associated with degenerative disc disease. Even when nerves function properly, chronic pain sensations are initiated through nerve cells. Thus, new ways of altering nerve cell function, as for example by stabilizing nerve cell wall membranes, may lead to new therapies for the treatment of pain.

Accordingly, embodiments of the invention may be used for applying a magnetic field which is particular to this device, to a nerve cell such that it alters nerve behavior. This invention relates to applying the magnetic field to the dorsal root ganglia by placing the device in the epidural space adjacent to the dorsal root. In this location, the catheter will modulate or suppress C-fiber firing. Modulation of C-fiber firing will intercept the pain pathway in both somatic and sympathetically mediated pain The helical design of the catheter allows impingement of the dorsal root regardless of the catheter's rotation.

Another object of the invention is to provide a magnetic device with a configuration of static magnets with a particular pole design that, when placed close to a nerve cell, alters the nerve cell's responses to external electrical stimuli. In order to achieve the result on nerve cells, it is necessary that the proximity of the individual magnets within the device be maintained such that each pole exerts a sphere of magnetic influence on the other poles in the device. Any alteration of the proximity of the magnets one to another, with alternating polarity, will change the effect or cause no effect upon the cell. Any alteration in the balance and symmetry of the power of each individual pole with respect to the other three poles will likewise impair the effect upon the cell

It is a further object of this invention to provide an alternating, quadripolar array in which each of the poles is in the shape of two cones joined at the directrix of the cone with the vertex of the two cones lying in a perpendicular axis of a circular directrix.

An additional object of this invention is to provide a device with four magnetic poles of alternating polarity in which the shape of the poles is in the form of two cones joined at the directrix with the vertex of the two cones lying in a perpendicular axis of a circular directrix of the cones such that the vertex of the cone shaped magnetic bodies may all be tilted toward the midline (on the side facing the animal body) such that the peak or vertex migration toward the midline will sharpen the angle of the field gradient.

It is further object of this invention to provide a device which contains a flux return ring on the back surface away from the body surface which is designed to return the magnetic flux thereby increasing the strength and gradient without altering the center charge symmetry and homogeneity of the 3 dimensional steep gradient field.

Another object of the invention is to provide a flux focusing ring surrounding the 4 static magnetic poles on the outer perimeter stationed midway between the top and bottom of the pole. Attachment means is provided to hold the focusing ring to the outer perimeter of the poles through the total support means.

It is a further object of the invention to provide a static magnetic pole of like polarity on the outer surface of each of the 4 poles of the invention such that the top of the core is oriented to the geometric side of the pole such that the axis of the two magnets form a 45° to 90° angle. The focusing magnet comprises a static magnetic pole.

It is a further object of this invention to control the focusing of each magnet with a smaller and weaker magnet than the primary pole such that the focusing ring containing magnets can focus and balance the symmetry of the therapeutic field

It will be apparent to those skilled in the art that the apparatus of this invention alters C-fiber firing both in vitro and in vivo.

Additional objects and advantages of the present invention will be set forth in part in the description that follows and, in part, will be apparent to those skilled in the art from the description or may be learned by practice of the invention.

### BRIEF SUMMARY OF THE INVENTION

In accordance with the principles of the present invention as embodied and as broadly described herein, a therapeutic static magnetic treatment device adapted for placement of at least four magnetic flux generator poles is applied such that they may be applied to the animal or human body as described for the various applications revealed herein. The device comprises a plurality of static magnetic bodies in each head of the applications, having at least two positive and two negative magnetic poles substantially in a single plane, the magnetic poles being oriented to define the four vertices of a quadrilateral shape, the two positive poles defining opposite diagonal vertices, and the two negative poles defining opposite diagonal vertices of the quadrilateral shape, each of the magnetic poles being magnetically attracted by the oppositely charger poles and being magnetically repelled by the like charged poles.

It is a further object of this invention to provide an alternating, quadripolar array in which each of the poles is in the shape of two cones joined at the directrix of the cone with the vertex of the two cones lying in a perpendicular axis of a circular directrix..

An additional object of this invention is to provide a device with four magnetic poles of alternating polarity in which the shape of the poles is in the form of two cones joined at the directrix with the vertex of the two cones lying in a perpendicular axis of a circular directrix of the cones such that the vertex of the cone shaped magnetic bodies may all be tilted toward the midline (on the side facing the animal body) such that the peak or vertex migration toward the midline will sharpen the angle of the field gradient.

It is further object of this invention to provide a device which contains a flux return ring on the back surface away from the body surface which is designed to return the magnetic flux thereby increasing the strength and gradient without altering the center charge symmetry and homogeneity of the 3 dimensional steep gradient field.

Another object of the invention is to provide a flux focusing ring surrounding the 4 static magnetic poles on the outer perimeter stationed midway between the top and bottom of the pole. Attachment means is provided to hold the focusing ring to the outer perimeter of the poles through the total support means.

It is a further object of the invention to provide a static magnetic pole of like polarity on the outer surface of each of the 4 poles of the invention such that the top of the core is oriented to the geometric side of the pole such that the axis of the two magnets form a 45° to 90° angle. The focusing magnet comprises a static magnetic pole.

It is a further object of this invention to control the focusing of each magnet with a smaller and weaker magnet than the primary pole such that the focusing ring containing magnets can focus and balance the symmetry of the therapeutic field.

An array of containment means, support means energizing means and control means of the embodiments will be presented for a variety of therapeutic purposes along with support data for the application.

### L. Potentiation of Epidural Anesthesia and Epidural Analgesia.

There is disclosed a method for suppressing nerve cell (particularly C-fiber) action potentials

A magnetic treatment device is placed at such a distance from a mammalian sensory neuron that the magnetic field of the treatment device reaches the sensory neuron. During such placement, and for a period thereafter, the nerve cell action potentials are suppressed. The magnetic treatment devices is comprised of four magnetic bodies having two positive and two negative magnetic poles substantially in a single plane, the magnet poles being oriented to define the four vertices of a quadrilateral shape, the two positive poles defining opposite diagonal vertices, and the two negative poles defining opposite diagonal vertices of the quadrilateral shape. Containment means art provided for holding the magnetic poles of the magnetic bodies in the quadrilateral orientation in the catheter by embedding the submicroscopic magnet devices in the wall of the catheter. The plurality of magnet bodies comprises four substantially identical cylindrical magnetic bodies, each having one magnetic center charge face. It is necessary that two of the cylindrical magnetic bodies have a positive magnetic pole on one face and two of the magnetic bodies have a negative magnetic pole on one face, and that the two positive and two negative magnetic poles on the magnetically charged faces of the four magnet bodies be in the quadrilateral orientation described above. The proximity of the individual magnets within the device must be maintained. Separation of the individual magnets or bringing the device in close proximity to other magnets will cause interference with the magnetic field and will change the flux lines and gradient of the field so that the device will not effectively alter the C-fiber firing.

### BRIEF DESCRIPTION OF THE DRAWINGS

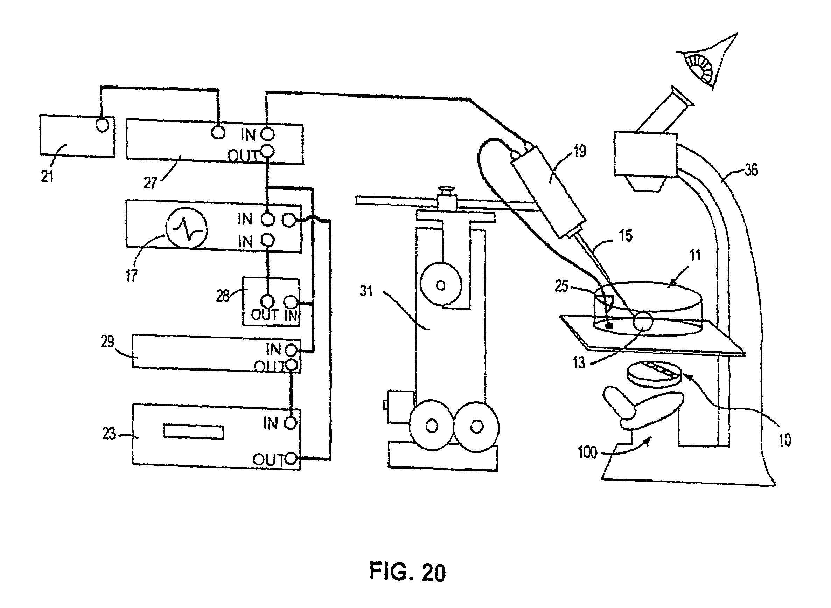

For a further understanding of the nature, objects, and advantages of the present invention, reference should be had to the following detailed description, read in conjunction with the following drawings, wherein like reference numerals denote like elements and wherein:

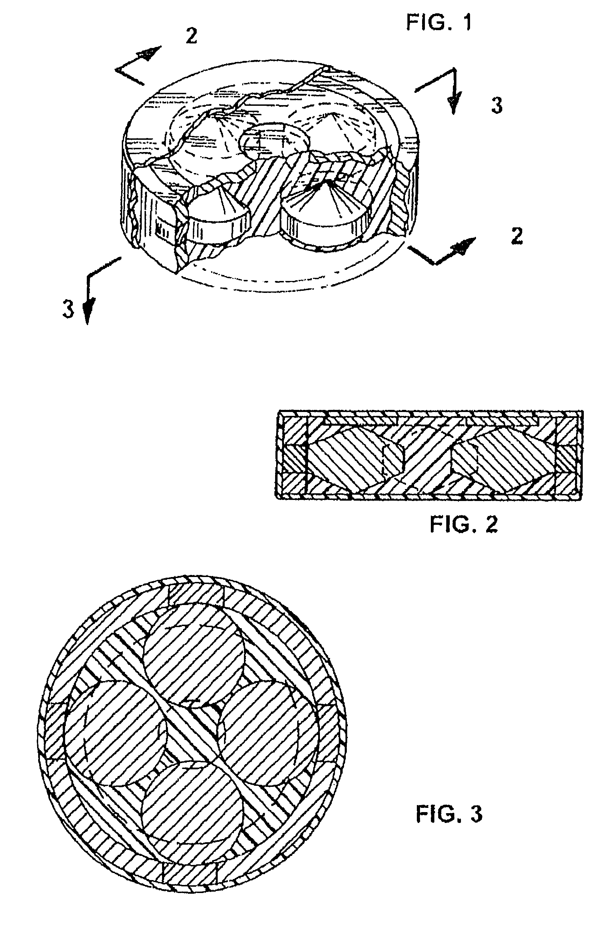

FIG. 1 is a perspective view of an embodiment of a magnetic device in accordance with the principles of the invention.
FIG. 2 is a cross section of the magnetic device in FIG 1 along the line 2--2
FIG. 3 is a plan view of the magnetic device in FIG. 1.

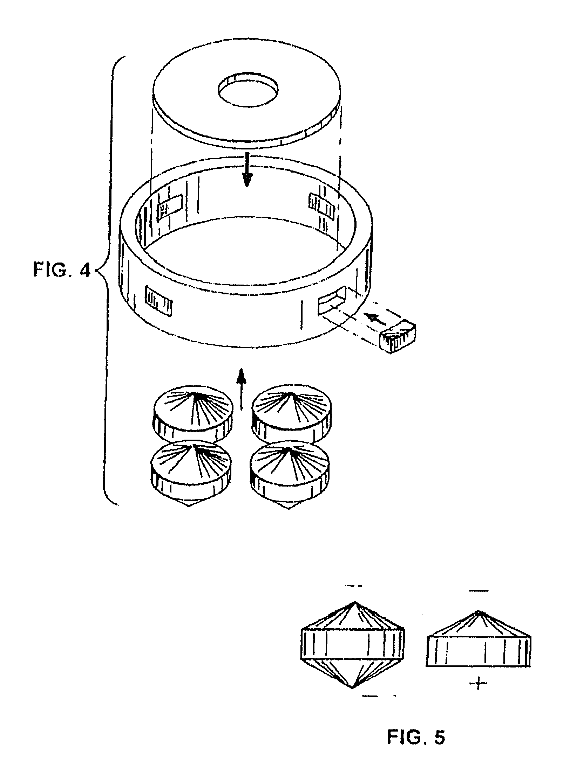

FIG. 4 is an exploded diagram of the magnetic device in FIG. 1
FIG. 5 is a side view of a "coned" magnet from the magnetic device in FIG. 1. Also included is a "single coned" magnet

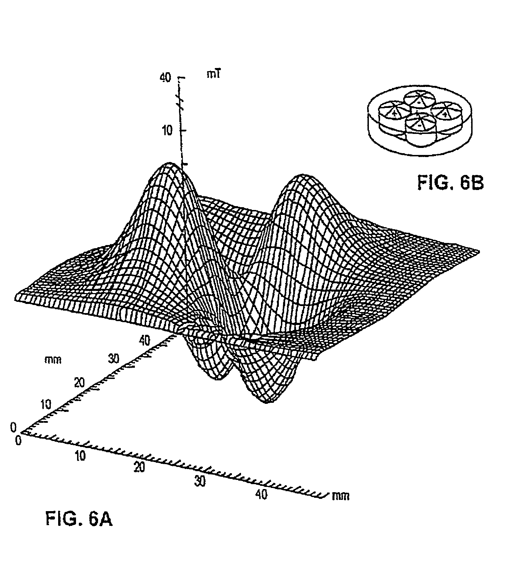

FIGS 6A-6B are illustrations of the magnetic field of magnetic device of FIG. 1, and another illustration of that device respectively.

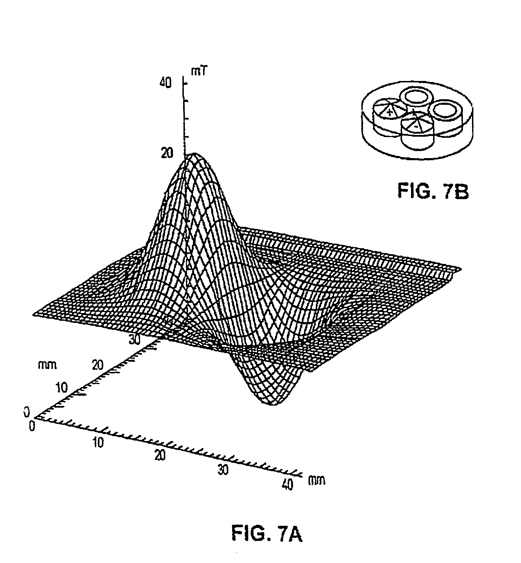

FIGS. 7A-7B are illustrations of the magnetic field of another magnetic device (with only two "coned" magnets) and an illustration of that device respectively.

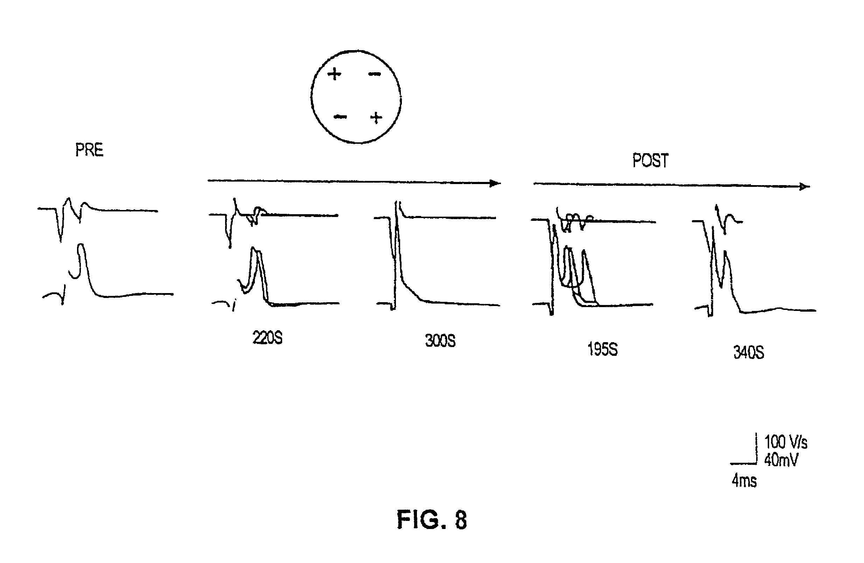

FIG. 8 shows oscilloscope traces from texts on mammalian nerve cells conducted using the magnetic device of FIG. 1.

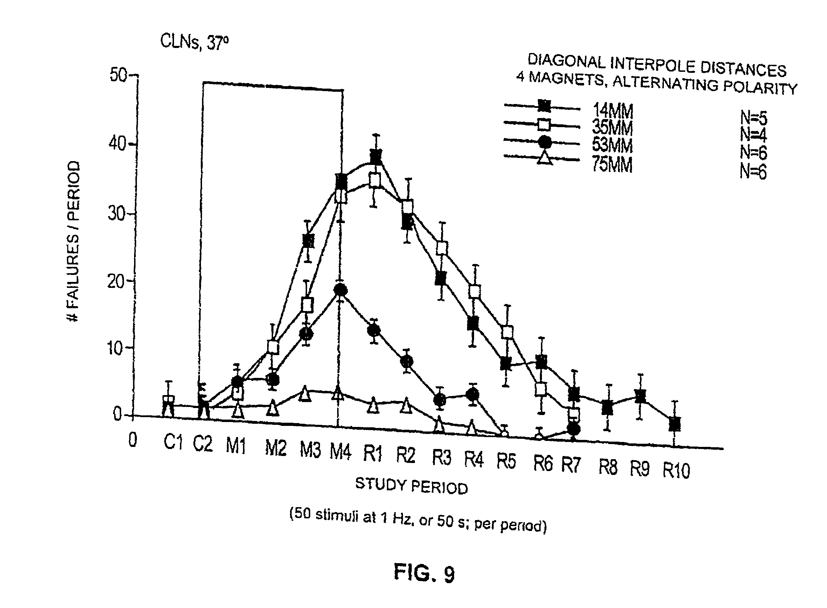

FIG. 9 is a graph comparing the average number of times neurons failed to elicit action potentials in response to applied stimuli before, during and after exposure to the magnetic device of FIG. 1 and to similar magnetic devices in which the magnets were spaced from each other at various distances

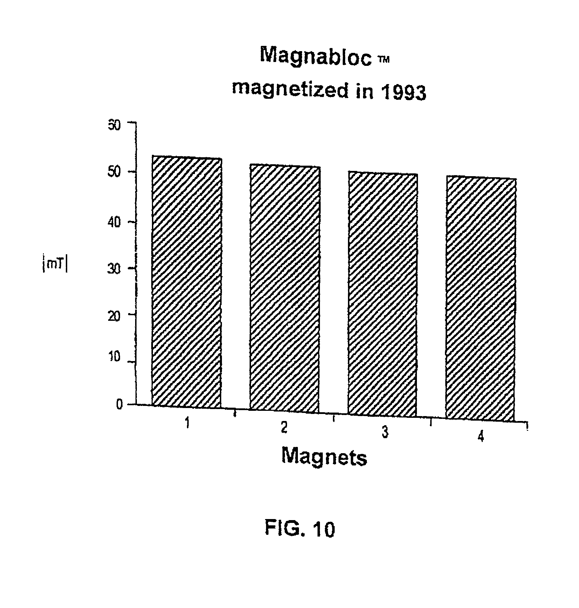

FIG. 10 is a bar graph representing the magnetic energy in milli Tesla (mT) as measured over the center of the pole in each of the 4 poles of a device as in FIG. 1 soon after it was manufactured in 1993.

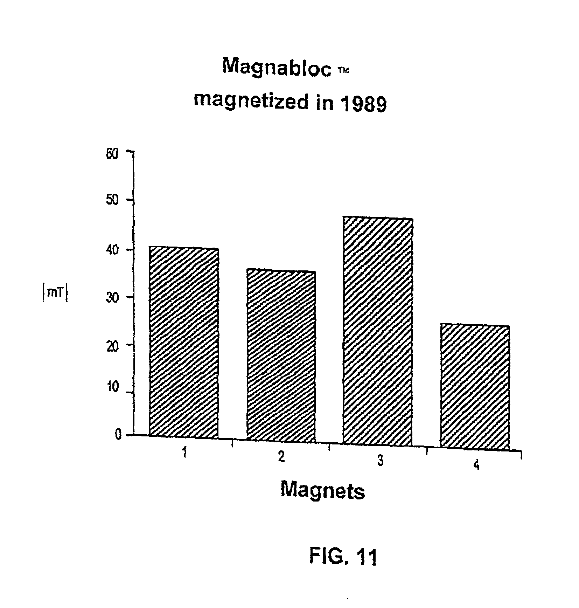

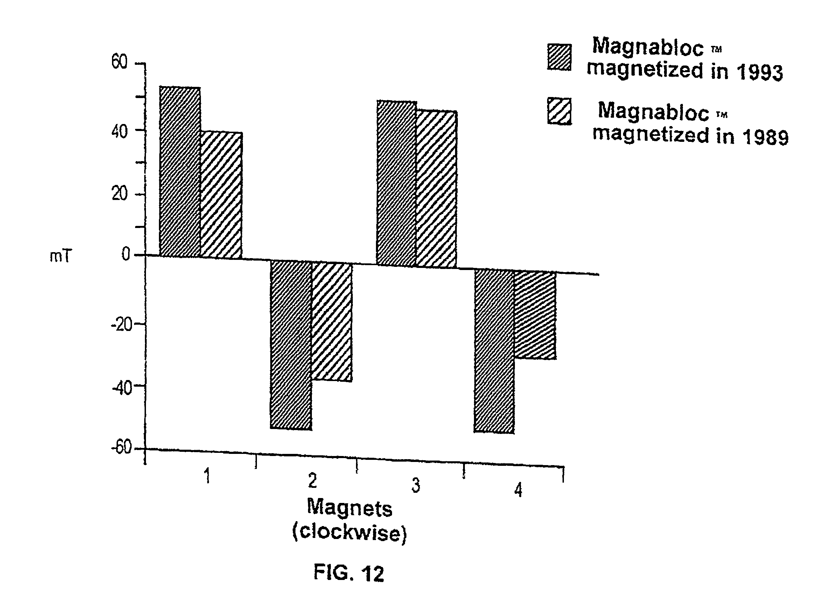

FIG. 11 is a bar graph representing the magnetic energy in milli Tesla (mT) as measured over the center of the pole in each of the 4 poles of a device as in FIG. 1 in 1993, the device being manufactured in 1989.
FIG.. 12 is a bar graph representing the magnetic energy in milli Tesla (mT) as measure in clockwise fashion over each pole of a device in FIG. 1 which was manufactured in 1989 and 1993--measurement was done in 1993.

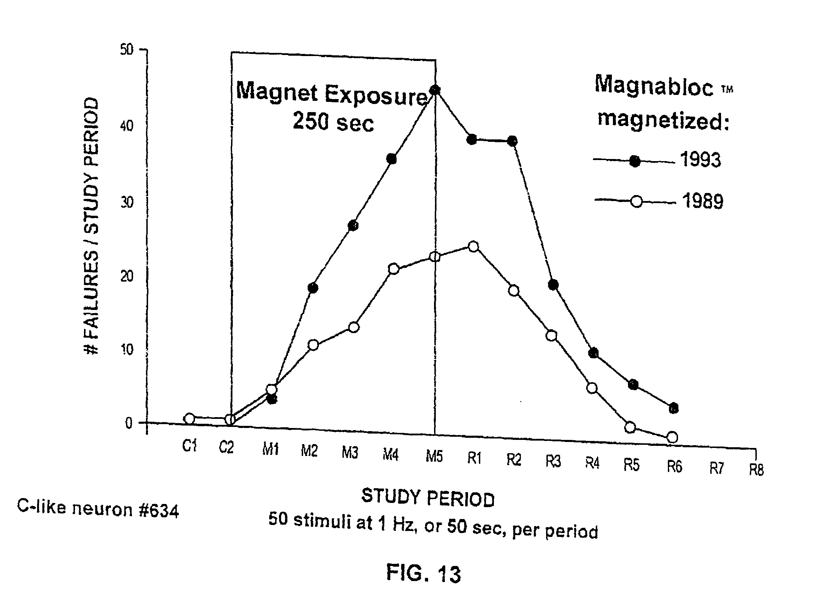

FIG. 13 is a graph comparing the average number of times neurons failed to elicit action potentials in response to applied stimuli during exposure to the magnetic device of FIG. 1 for devices manufactured in 1989 (50% loss of energy in one pole) and 1993 (full energy in each of 4 poles).
F. Potentiation of Epidural Anesthesia and Epidural Analgesia
FIG. 20 is a side view of an experimental design used to monitor the suppression of nerve cell action potentials with the method of the present invention.

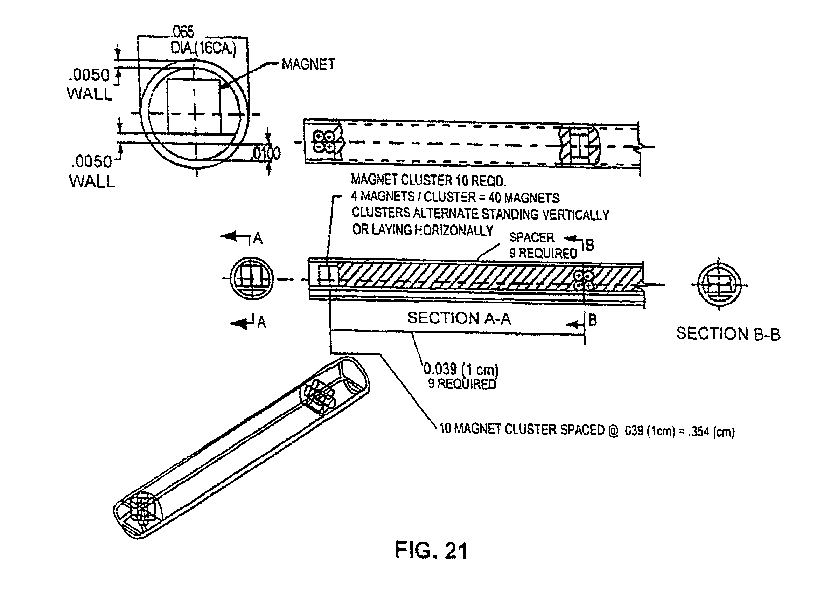

FIG. 21 is a plan view of the epidural catheter that may be used in applying the method of the intervention.

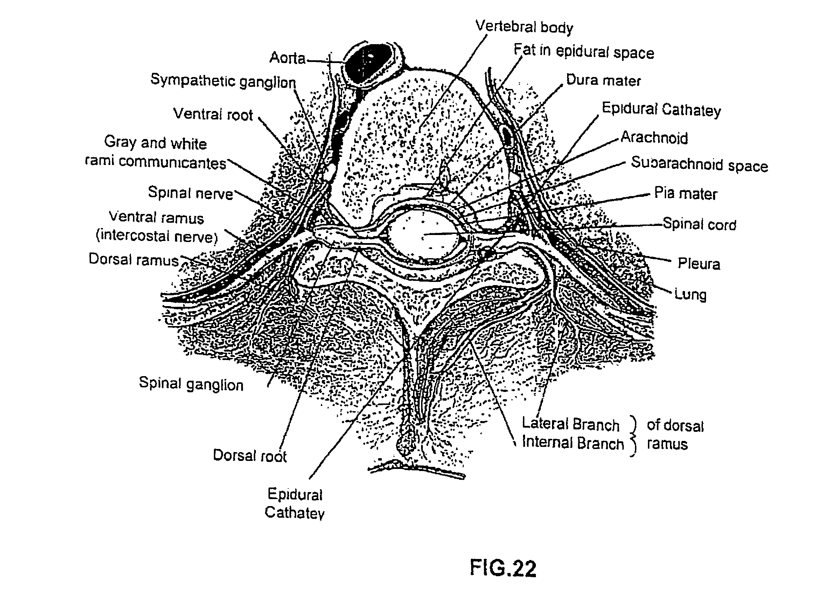

FIG. 22 is a cross-sectional view through a thoracic vertebrae, showing placement of the epidural catheter.

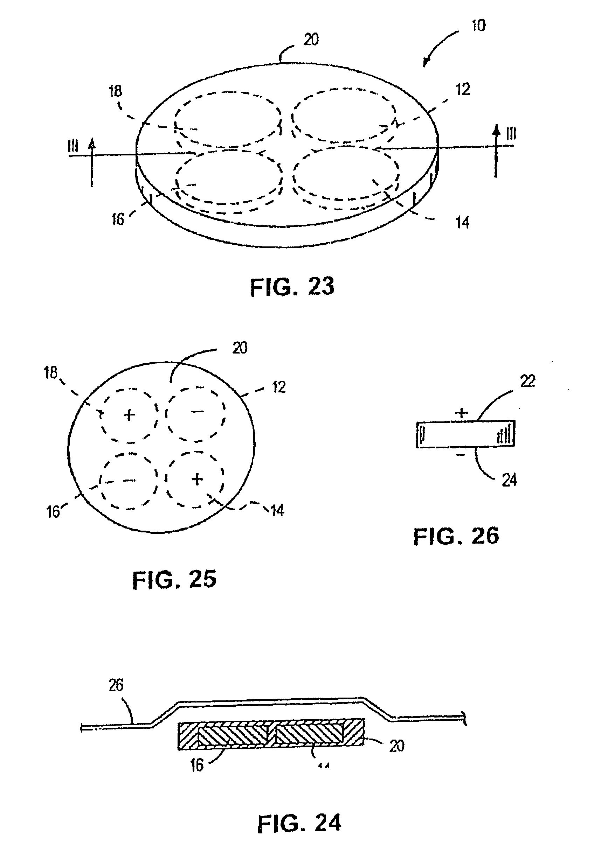

FIG. 23 is a perspective view of an embodiment of a magnetic device without "cones" in accordance with the principles of the invention.
FIG. 24 is a cross section of the magnetic device in FIG. 23 along the line III-III.
FIG. 25 is a plan view of the magnetic device in FIG. 23.
FIG. 26 is a side view of a magnet (without cones) from the magnetic device in FIG. 23.

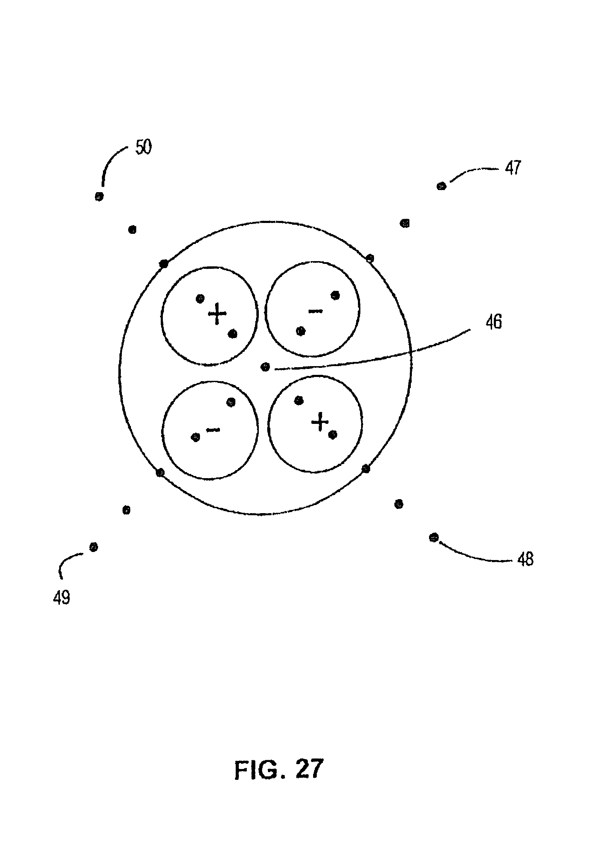

FIG. 27 is a top view of the magnetic device of FIG. 23, with dots added to designate surface magnetic field measurement locations.

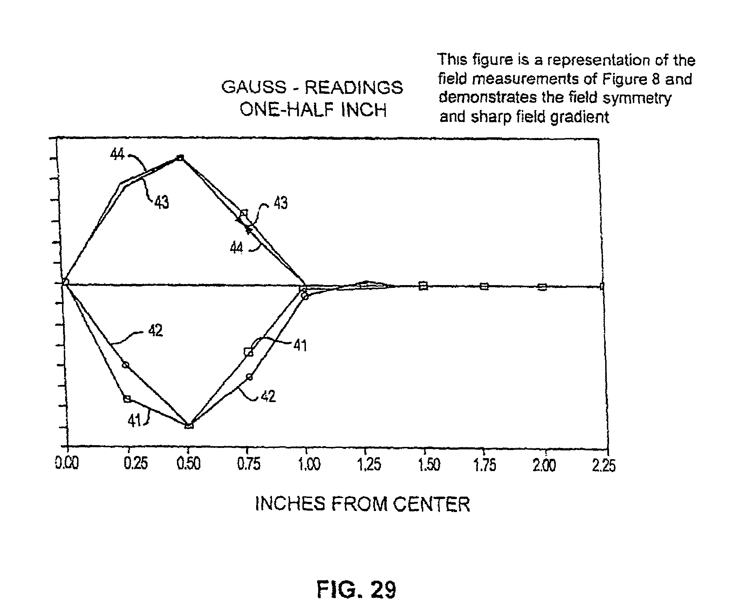

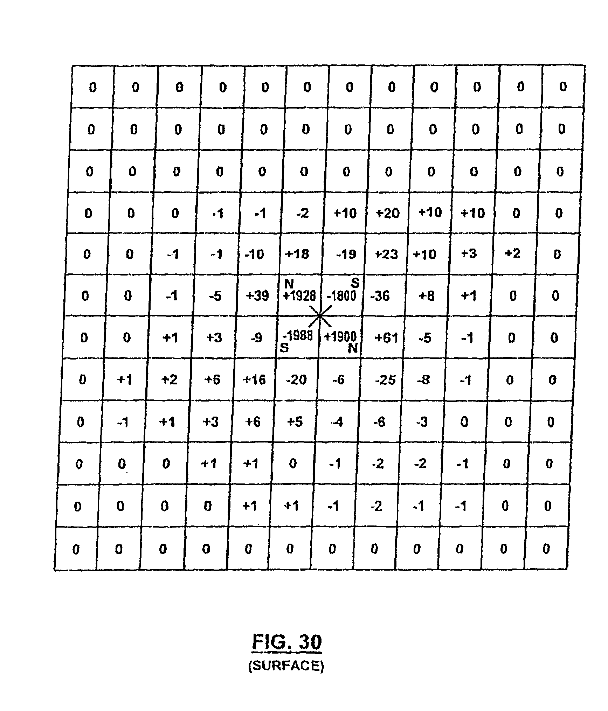

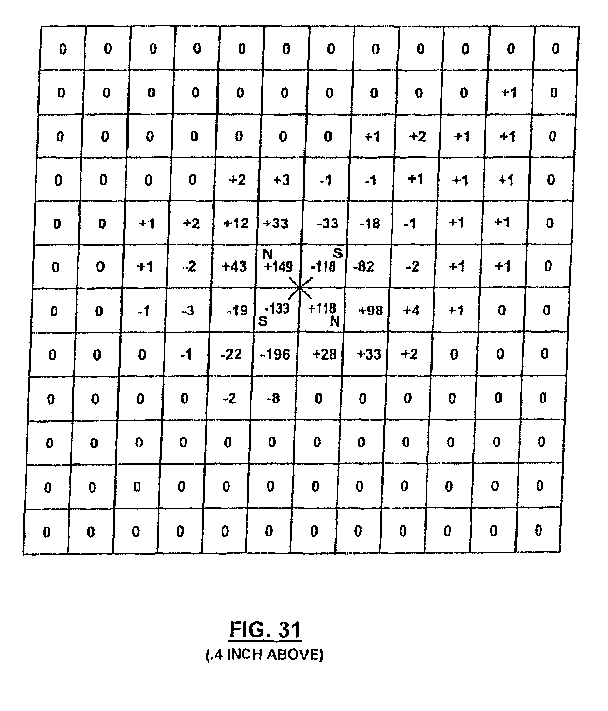

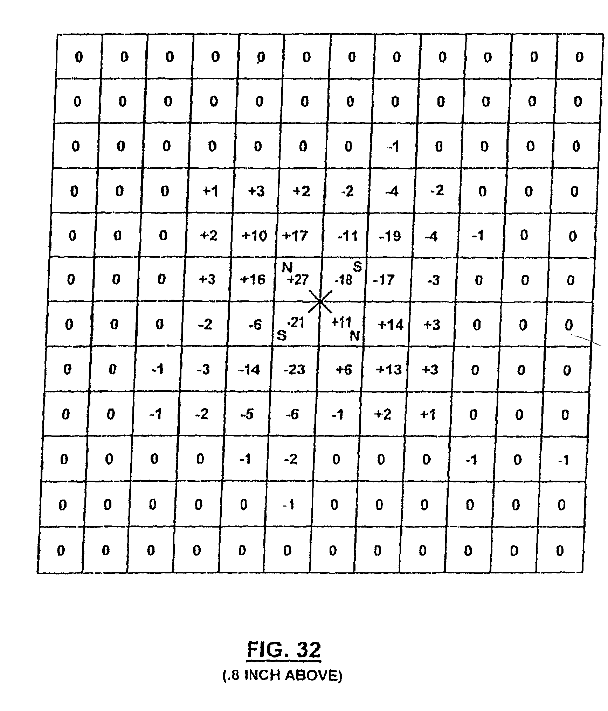

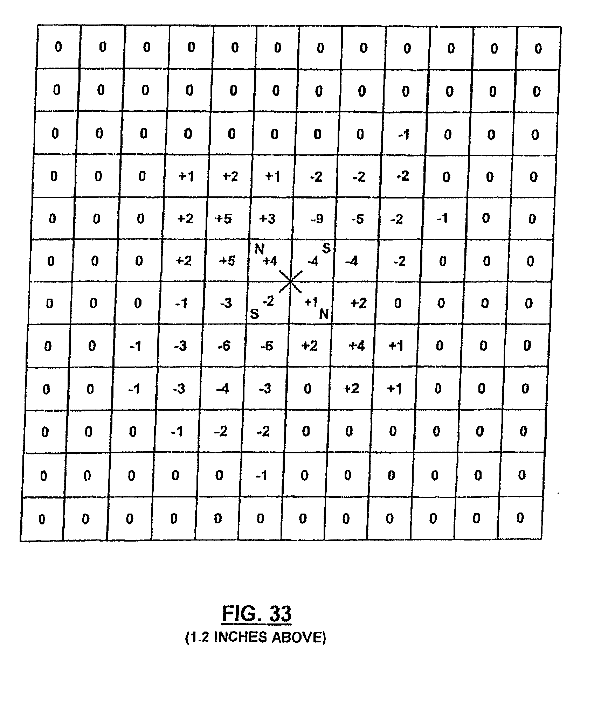

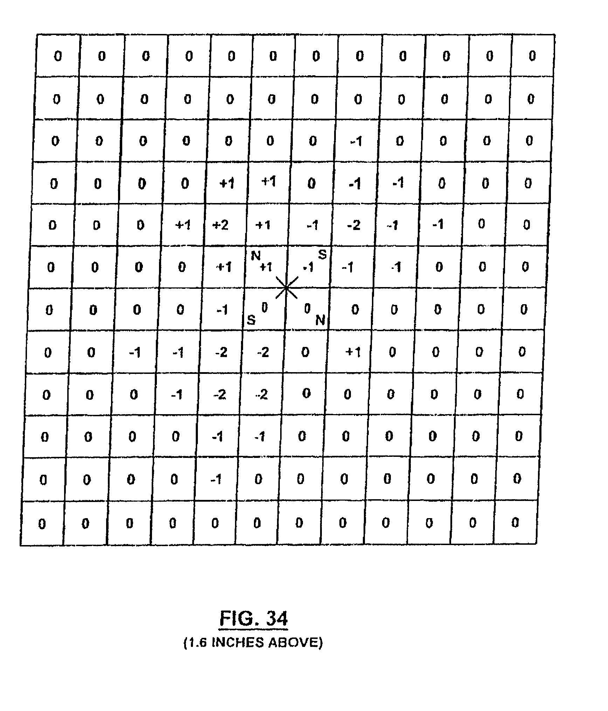

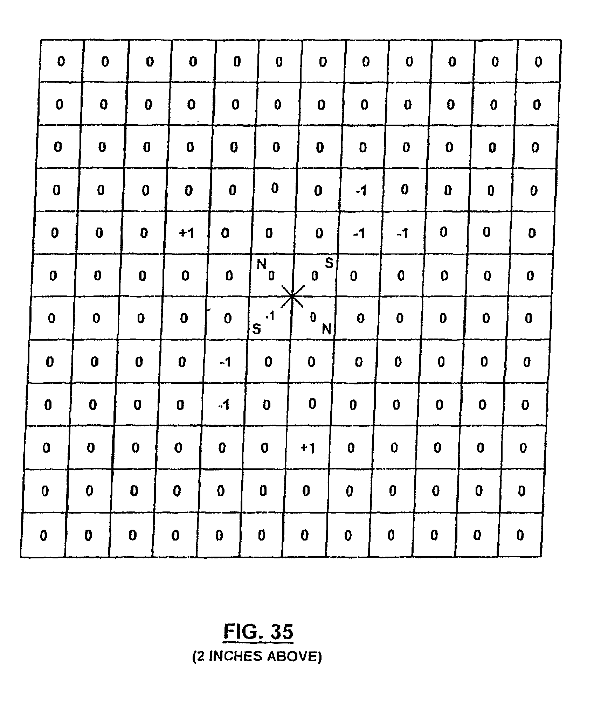

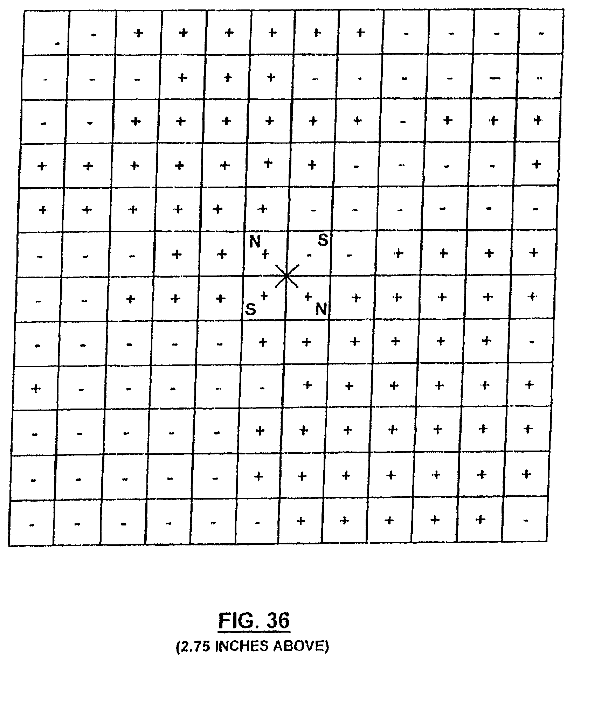

FIG. 29 is a graph of magnetic field measurements taken at the measurement locations designated in FIG. 27.
FIGS..30-.36 are grids of magnetic field measurements taken at various distances from the surface of the magnetic device of FIG. 23.

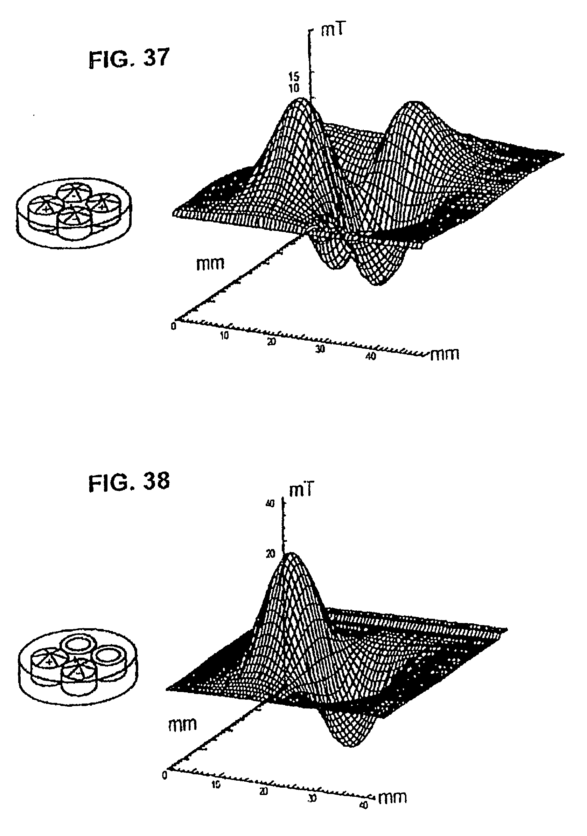

FIG. 37 is a magnetic field plot for the magnetic device of FIG. 23
FIG. 38 is a magnetic field plot for a magnetic device having one positive and one negative magnetic pole.

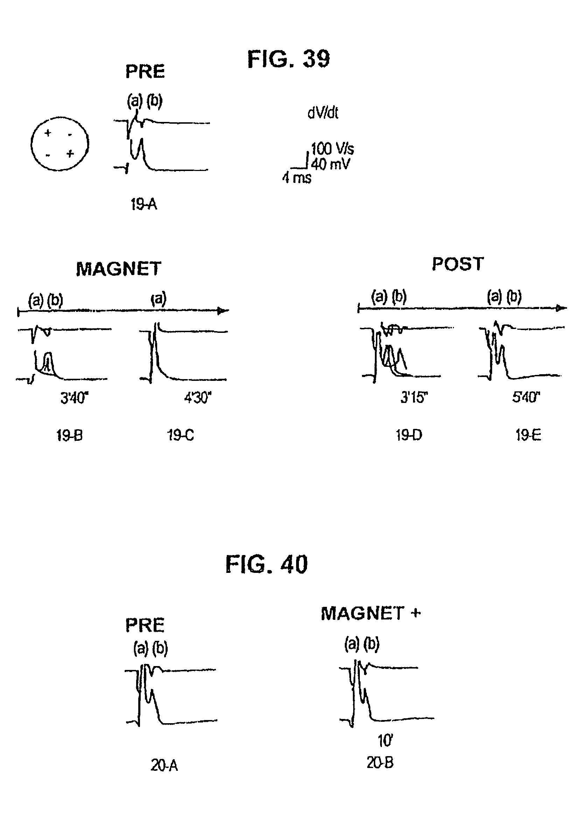

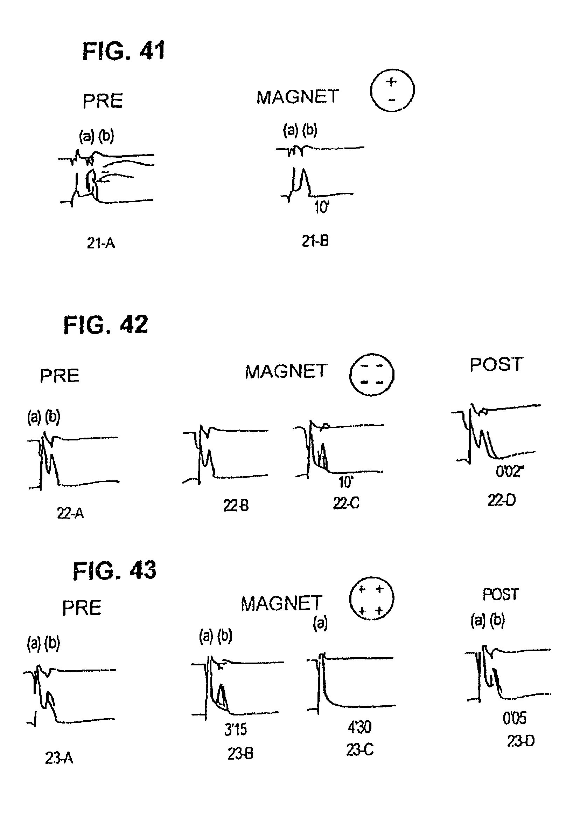

FIGS. 39-43 are a series of diagrams showing the output on an oscilloscope connected to an electric probe inserted in a mammalian nerve cell 3

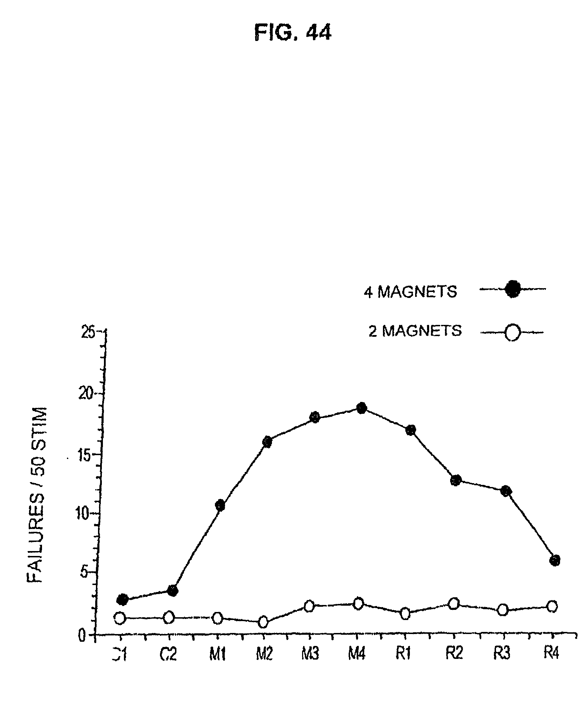

FIG. 44 is a graph showing average number of times neurons failed to elicit action potentials in response to stimuli applied as described in Example No. 9.

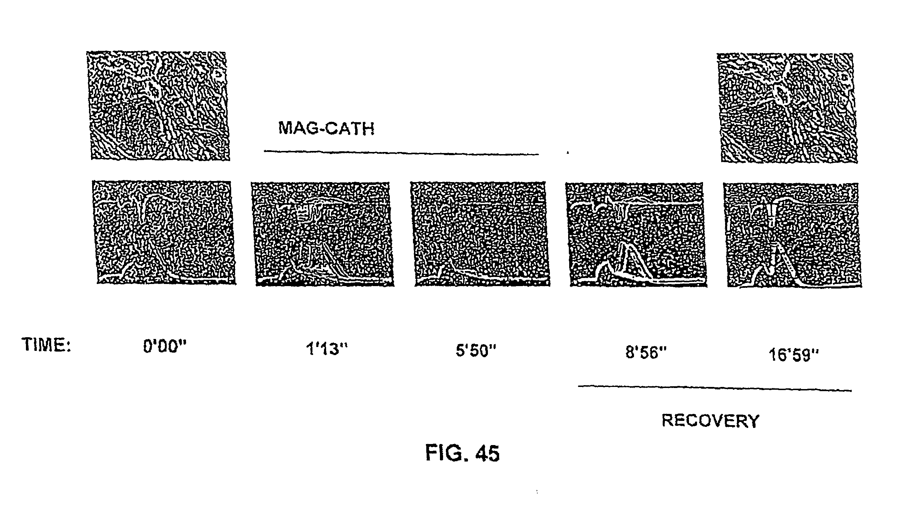

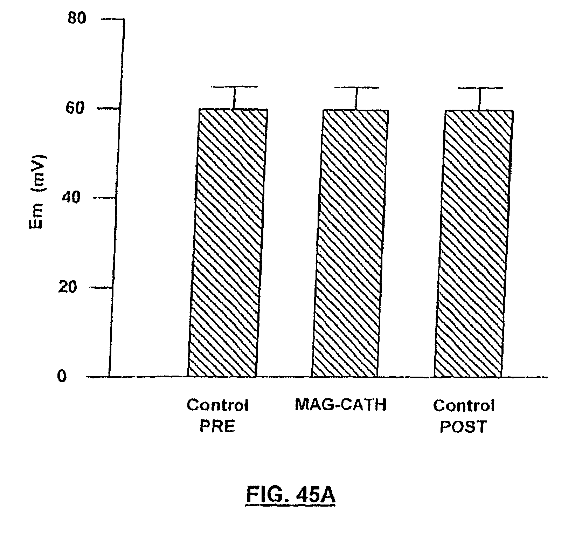

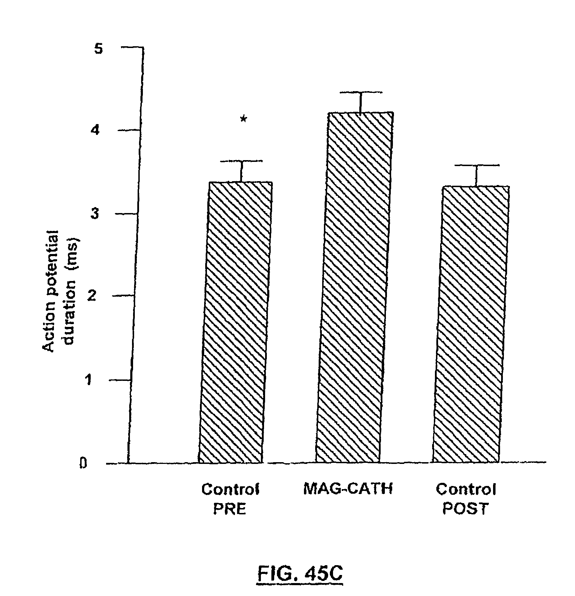

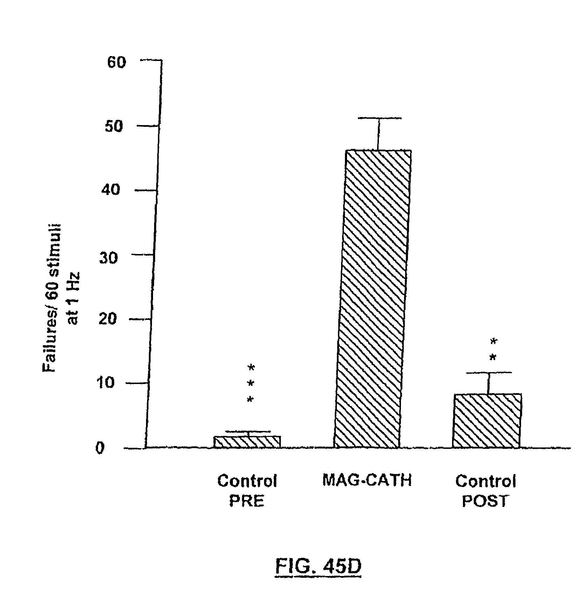

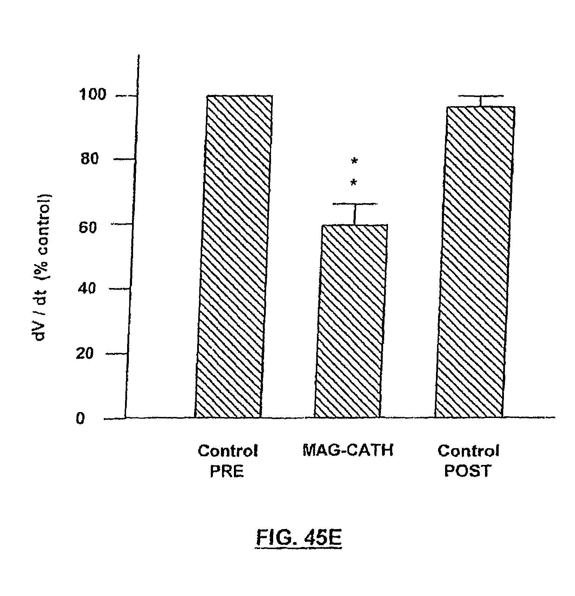

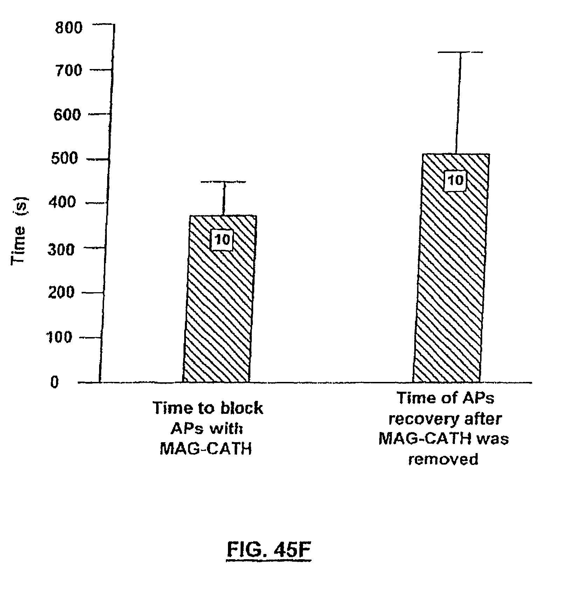

FIG. 45 is a photographic series showing the output on an oscilloscope connected to an electric probe inserted in a mammalian C-fiber in tissue culture; both before, during and after exposure to the magnetic field generated by the epidural Magna Cath.TM., This recording clearly shows that the Magna Cath.TM. reversibly blocks C-fiber firing in vitro.
FIG. 45A demonstrates that the Magna Cath.TM. doesn't alter the electronics.
FIG. 45B demonstrates that the Magna Cath.TM. significantly alters the action potential latency of C-fibers when quantitatively compared to control before and after treatment.
FIG. 45C demonstrates the prolonged action potential duration of C-fibers when exposed to the Magna Cath.TM.
FIG. 45D depicts dramatic failure of action potentials on a quantitative basis when stimulated at 1 Hz and exposed to the Magna Cath.TM,.
FIG. 45E reveals a significant change in the dv/dt when C-fibers are exposed to the Magna Cath.TM., This change is related to change in sodium currents.
FIG. 45F represents the time required to block and for recovery of C-fiber action potential.

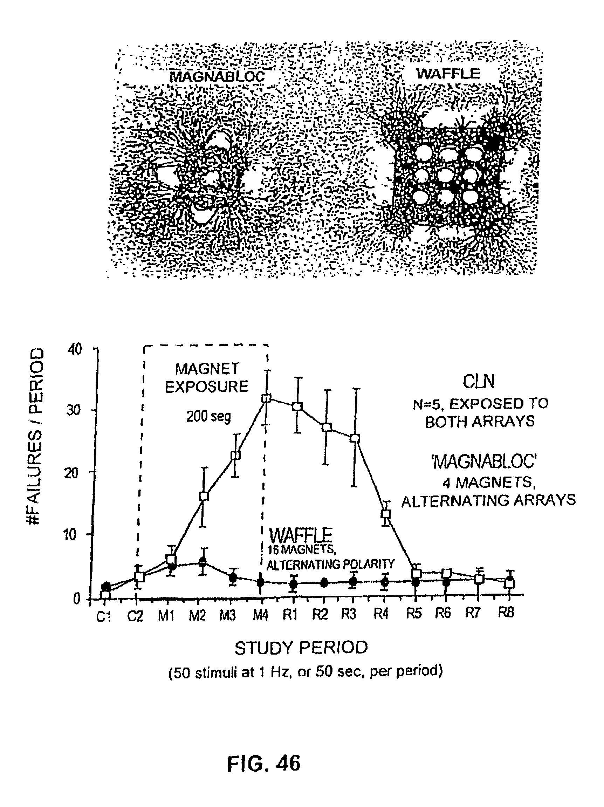

FIG. 46 illustrates a magnetic field around devices and comparison failures between Magna Bloc.TM. and a 16 array of magnets.

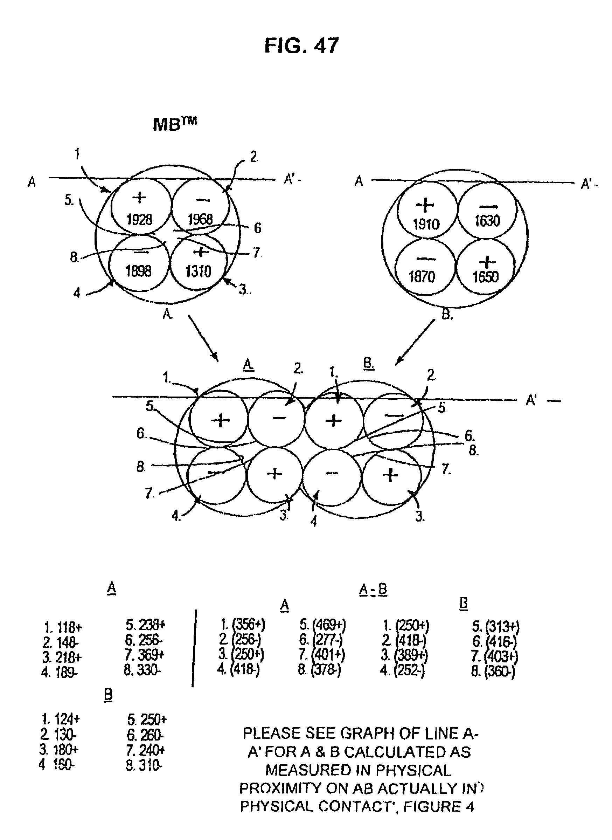

FIG. 47 shows the arrangement of two magnetic devices and the measurement of their magnetic field.

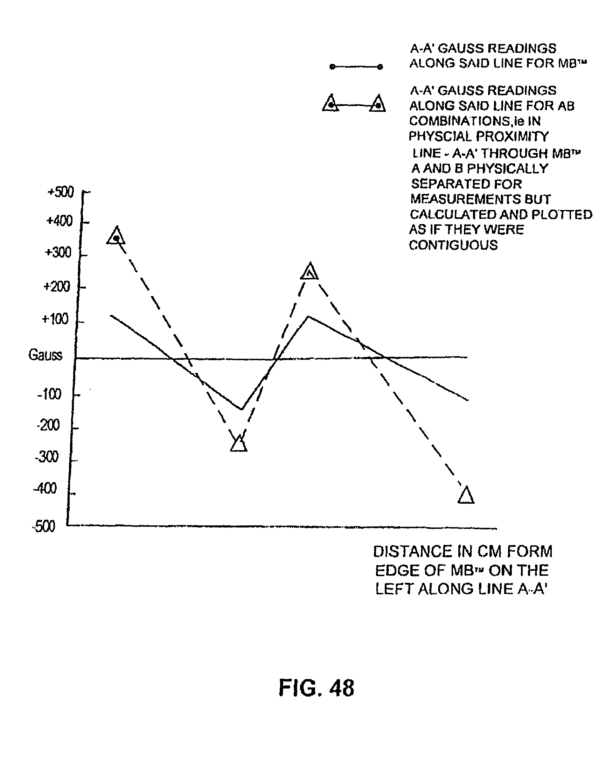

FIG. 48 is a graph showing the magnetic field for devices of FIG. 47.

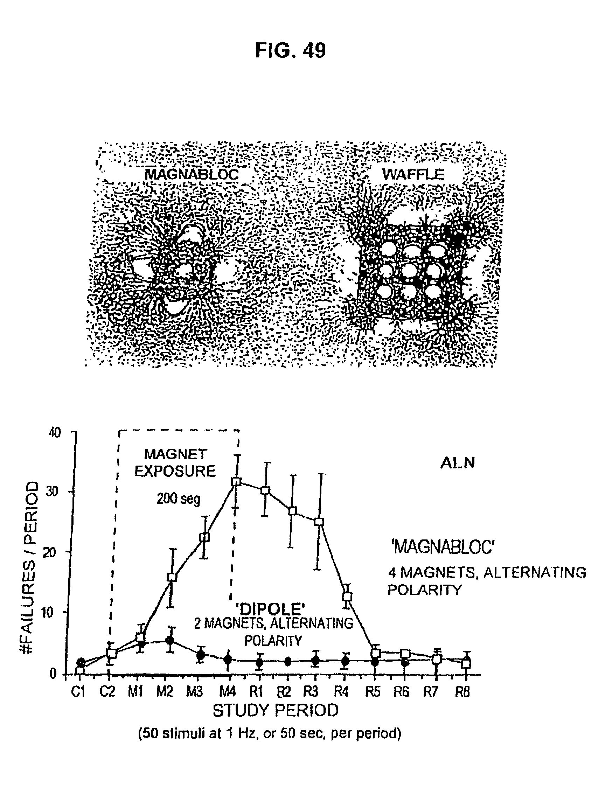

FIG. 49 illustrates the field around the device and a two magnet device, and shows comparative neurons firing data for the two devices.

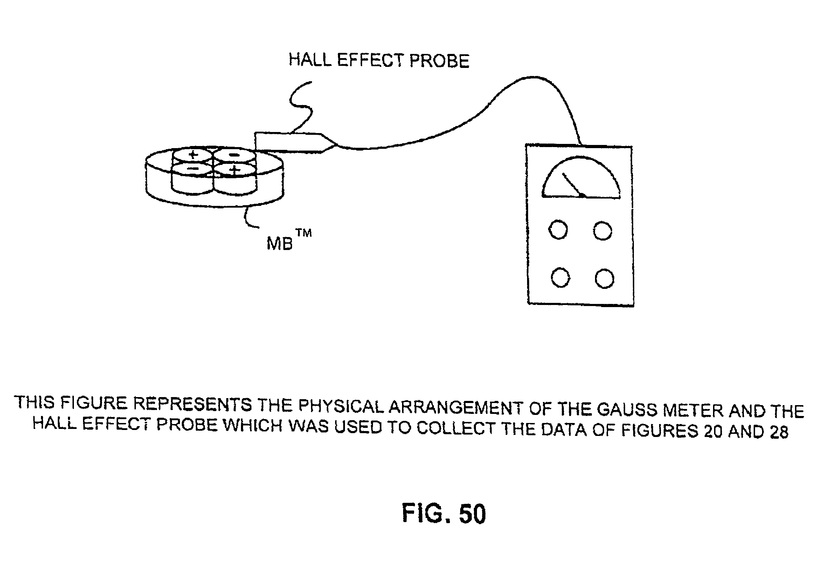

FIG. 50 schematically illustrates the apparatus used to measure the magnetic field data in FIGS. 47 and 48.

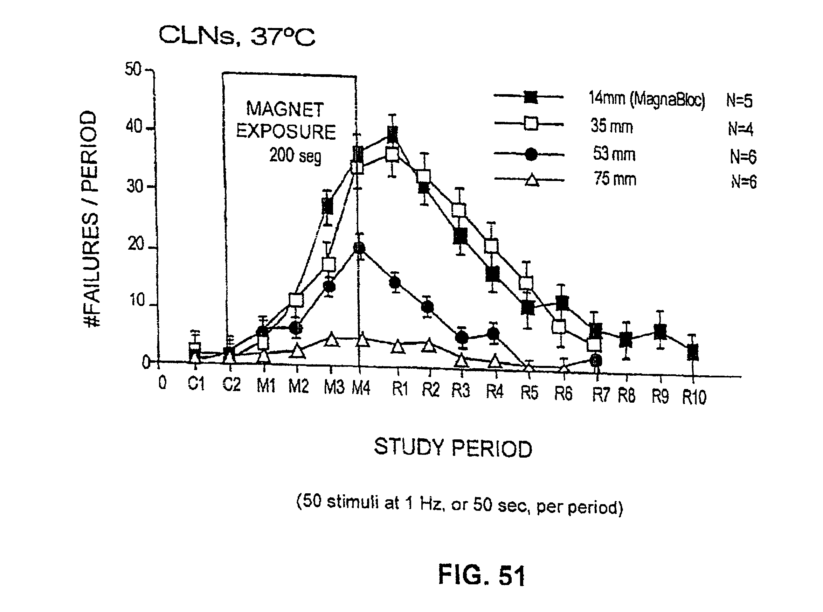

FIG. 51 is a graph comparing the average number of times neurons failed to elicit action potentials in response to applied stimuli before, during and after exposure to the magnetic device of FIG. 23 and to similar magnetic devices in which the magnets were spaced from each other at various distances.

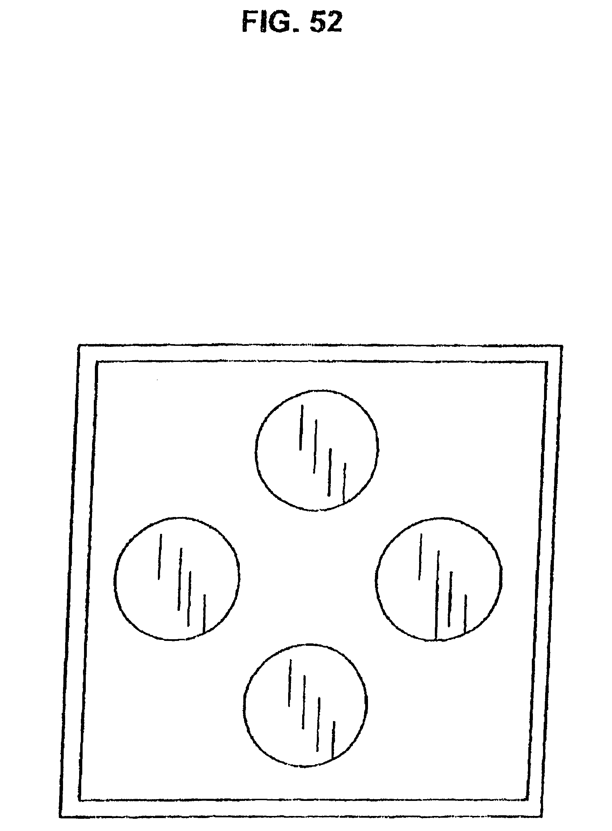

FIG. 52 shows a test fixture in which the magnets were held during the testing for which the data are shown in FIG. 51.

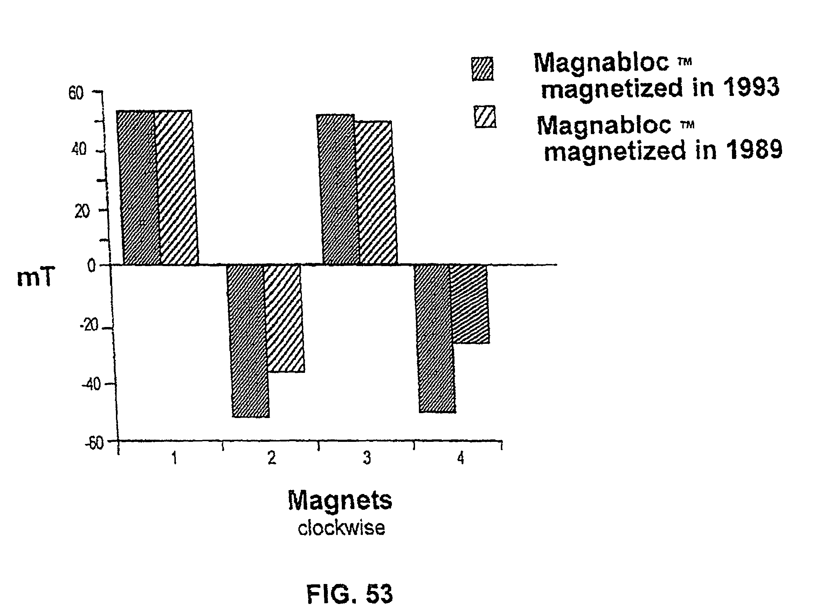

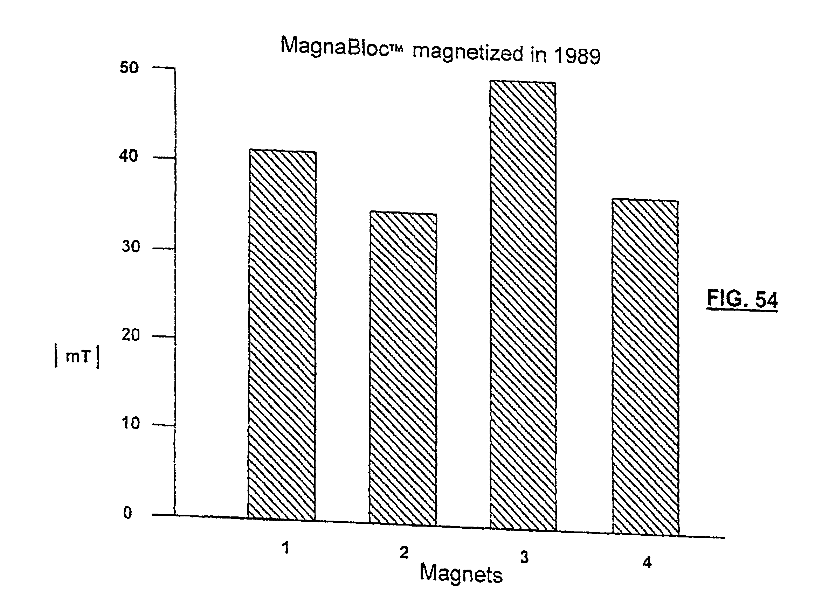

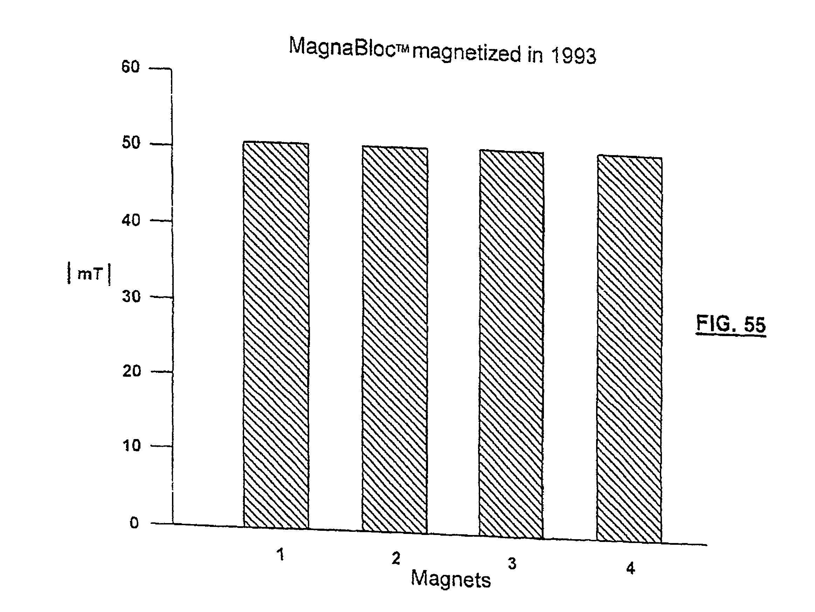

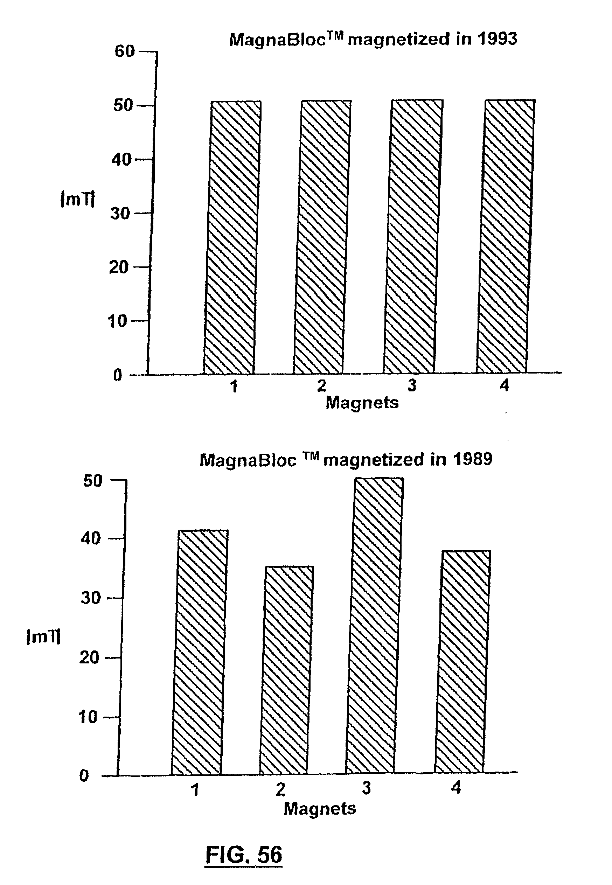

FIGS. 53-56 are illustrations and graphs demonstrating the difference in pole strengths and a comparison of their effectiveness on blocking C-fibers.

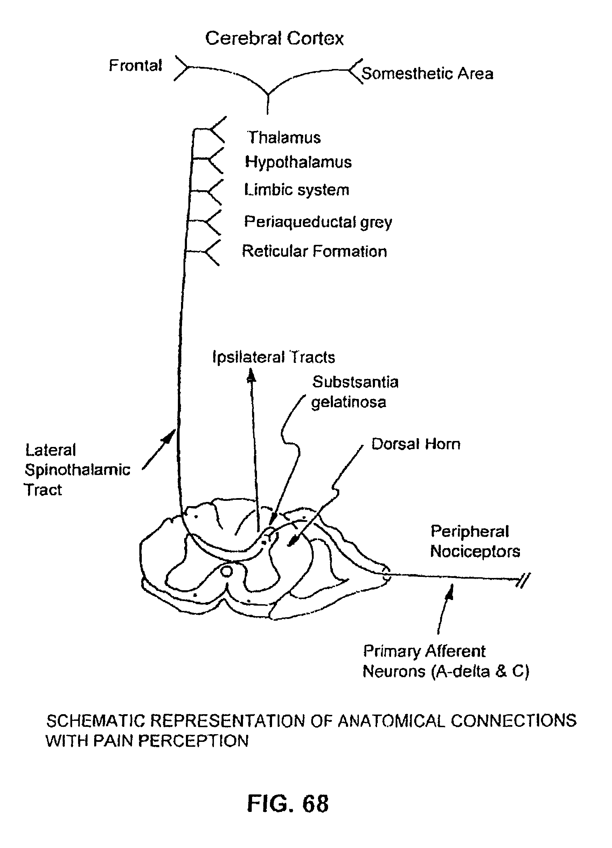

FIG. 68 Schematic representation of anatomical connections associated with pain perception.

The Figures referenced above illustrate the preferred embodiment of the present invention which define ein apparatus for altering the charge distribution upon living membranes.

Furthermore, there is taught an apparatus for altering the charge distribution upon living membranes with functional stabilization of the membrane physical electrical integrity.

Further, there is taught an apparatus for altering the stability of excitable membranes and other charged structures and systems in order to treat ailments of animals.

Further, there is taught an apparatus to provide a static magnetic device for production of a magnetic field for treatment of disorders.

There is further taught an apparatus to provide a static magnetic device for production of a magnetic field for treatment of disorders wherein such a device being powered by a particular static magnetic field and having, inter alia, an alternating polarity, quadripolar array which generates a 3 dimensional, steep field gradient (greater than 0.25mt/mm) with a homogenous field.

Furthermore, there is taught an apparatus to provide a static magnetic device for production of a magnetic field for treatment of disorders according to claim 4 wherein an alternating, quadripolar array in which each of the poles is in the shape of two cones joined at the derectrix of the cone with the vertex of the two cones lying in a perpendicular axis of a circular directrix is provided..

There is further taught an apparatus to provide a static magnetic device for production of a magnetic field for treatment of disorders wherein a device contains four magnetic poles of alternating polarity in which the shape of the poles is in the form of two cones with the directrix with the vertex of the two cones lying in a perpendicular axis of a circular directrix of the cones such that the vertex of the cone shaped magnetic bodies may all be tilted toward (or away from) the midline (on the side facing the animal body) such that the peak or verlex migration toward the midline will vary the field gradient

There is further taught an apparatus to provide a static magnetic device for production of a magnetic field for treatment of disorders wherein a device contains a flux return ring on the back surface away from the body surface which is designed to return the magnetic flux thereby altering the strength and gradient without materially altering the center charge symmetry and homogeneity of the 3 dimensional steep gradient field which is in contact with the animal body.

Another feature that is taught is an apparatus to provide a static magnetic device for production of a magnetic field for treatment of disorders wherein a device contains a flux focusing ring surrounding the 4 magnetic poles on the outer perimeter stationed substantially midway between the top and bottom of the pole. Attachment means is provided to hold the focusing ring to the outer perimeter of the poles throughout the total support means.

There is further taught an apparatus to provide a static magnetic device for production of a magnetic field for treatment of disorders wherein the device provides a static magnetic pole of like polarity on the outer surface of the flux focusing ring adjacent to each of the 4 poles of the invention (focusing magnet) such that the end or top of the focusing magnet is oriented to the geometric side of the pole such that the axis of the two magnets form a 45 to 90 degree angle. The focusing magnet comprises a static magnetic pole. The angle of the focusing magnet is such that the axis forms a 45 to 90 degree angle on the body surface side and a 90 to 135 degree to the surface away from the body.

Further, there is taught an apparatus to provide a static magnetic device for production of a magnetic field for treatment of disorders
wherein it is a further object of the invention to control the focusing of each magnet with a smaller and weaker magnet than the primary pole such that the focusing ring containing magnets can focus and balance the symmetry of the therapeutic field.

There is further taught an array of embodiments, for a variety of therapeutic purposes.

There is further taught the proper placement of the magnetic devices for the currently known therapeutic uses.

There is further taught a method of design and manufacture of an inexpensive, center charged and homogeneous static magnetic flux magnet.

There is further taught the addition of a plastic material to a permanent magnet amalgam along with a proprietary silica colloid and curing the amalgam under the influence of a strong, center charged homogenous magnetic field.

There is further taught the application of the quadripolar array in a microscopic embodiment which is housed in a catheter designed to be placed in the epidural space and other body cavities such that the magnetic quadripolar flux generators are arranged in a helix such that migration or rotation of the device will not alter the efficacy of the therapeutic device.

There is further provided the device which provides an alternating, quadripolar array in which each of the poles is in the shape of two cones joined at the directrix of the cone with the vertex of the two cones lying in a perpendicular axis of a circular directrix.

There is further provided the device which provides a device with four magnetic poles of alternating polarity in which the shape of the poles is in the form of two cones joined at the directrix with the vertex of the two cones lying in a perpendicular axis of a circular directrix of the cones such that the vertex of the cone shaped magnetic bodies may all be tilted toward the midline (on the side facing the animal body) such that the peak or vertex migration toward the midline will alter the field gradient.

There is further taught the device which contains a flux return ring on the back surface away from the body surface which is designed to return the magnetic flux thereby increasing the strength and gradient without altering the center charge symmetry and homogeneity of the 3 dimensional steep gradient field.

There is further taught the device which provides a flux focusing ring surrounding the 4 static magnetic poles on the outer perimeter stationed essentially midway between the top and bottom of the pole. Attachment means is provided to hold the focusing ring to the outer perimeter of the poles through the total support mean.

There is further taught the device which provides a static magnetic pole of like polarity on the outer surface of each of the 4 poles of the invention such that the top of the core is oriented to the geometric side of the pole such that the axis of the two magnets form a 45 to 90 degree angle. The focusing magnet comprises a static magnetic pole.

There is further taught the device which provides control of the focusing of each magnet with a smaller and weaker magnet than the primary pole such that the focusing ring containing magnets can focus and balance the symmetry of the therapeutic field.

There is further taught the methods, devices, techniques, and processes which relate specifically to potentiation of epidural anesthesia and epidural analgesia.

There is further taught he methods, devices, techniques, and processes which specifically relates to a method for using submicroscopic, quadripolar, circular, center charged, energy balanced magnetic device in a four (4) magnet array of alternating polarity in which the magnetic poles are separated only by a distance which will allow a magnetic sphere of influence on all adjacent poles to suppress the firing of action potentials of mammalian sensory neurons.

There is further taught the method which provides for applying a magnetic field which is particular to this device, to a nerve cell such that it alters nerve behavior.

There is further taught the method which provides applying the magnetic field to the dorsal root ganglia by placing the device in the epidural space adjacent to the dorsal root.

There is further disclosed the method which provides for a catheter to modulate or suppress C-fiber firing.

There is further disclosed method which provides for the helical design of the catheter so as to allow impingement of the dorsal root regardless of the catheter's rotation.

There is further disclosed a magnetic device with a configuration of static magnets with a particular pole design that, when placed close to a nerve cell, alters the nerve cell's responses to external electrical stimuli.

There is further taught an alternating, quadripolar array in which each of the poles is in the shape of two cones joined at the directrix of the cone with the vertex of the two cones lying in a perpendicular axis of a circular directrix.

There is further taught a device with four magnetic poles of alternating polarity in which the shape of the poles is in the form of two cones joined at the directrix with the vertex of the two cones lying in a perpendicular axis of a circular directrix of the cones such that the vertex of the cone shaped magnetic bodies may all be tilted toward the midline (on the side facing the animal body) such that the peak or vertex migration toward the midline will sharpen the angle of the field gradient.

There is further taught a device which contains a flux return ring on the back surface away from the body surface which is designed to return the magnetic flux thereby increasing the strength and gradient without altering the center charge symmetry and homogeneity of the 3 dimensional steep gradient field.

There is further taught a flux focusing ring surrounding the 4 static magnetic poles on the outer perimeter stationed midway between the top and bottom of the pole.

There is further taught the device wherein the attachment means is provided to hold the focusing ring to the outer perimeter of the poles through the total support means.

There is further taught static magnetic pole of like polarity on the outer surface of each of the 4 poles of the invention such that the top of the core is oriented to the geometric side of the pole such that the cods of the two magnets form a 45° to 90° angle. The focusing magnet comprises a static magnetic pole.

There is further taught the method which provides for control of the focusing of each magnet with a smaller and weaker magnet than the primary pole such that the focusing ring containing magnets can focus and balance the symmetry of the therapeutic field.

## Claims

1. A system for altering the charge distribution upon living membranes with functional stabilization of the physical electrical integrity of the membranes, the system comprising:
(a) a quadripolar, circular, centre charged, energy balanced magnetic device in a four magnet array of alternating polarity in which the magnetic poles are separated only by a distance which allows a magnetic sphere of influence on all adjacent poles to modulate or suppress the firing of action potentials of mammalian sensory neurons;
(b) a flux focusing ring surrounding the four magnet array on its outer perimeter stationed essentially midway between the top and bottom of the magnetic poles; and
(c) a flux return ring away from the membrane surface for returning the magnetic flux, thereby altering the strength and gradient without altering centre charge symmetry and homogeneity of the three-dimensional steep gradient field which is in contact with the membrane;
**characterised in that.**
(d) the system includes an epidural catheter; and
(e) the magnetic device is suitable to be placed in said epidural catheter.

2. A system as claimed in claim 1, wherein the system provides a static magnetic device for production of a magnetic field for treatment of various disorders.

3. A system as claimed in claim 2, wherein the system provides a static magnetic pole of like polarity on the outer surface of the flux focusing ring adjacent to the geometric side of the poles of the magnets such that the static magnetic pole is oriented to the geometric sides of the poles of the magnets at an angle of 45 to 90 degrees.

4. A system as claimed in any preceding claim, comprising an alternating, quadripolar array in which each of the poles is in the shape of two cones joined at the directrix of the cone with the vertex of the two cones lying in a perpendicular axis of circular directrix.

5. A system as claimed in any preceding claim, wherein the magnets are configured to generate a three-dimensional, steep gradient of at least 0.25mT/mm.

6. A system as claimed in any preceding claim, comprising a plurality of magnetic devices arranged in a helical configuration along the catheter.

## Patentansprüche

1. System zur Veränderung der Ladungsverteilung auf lebenden Membranen mit einer funktionellen Stabilisierung der physikalischen elektrischen Integrität der Membranen, wobei das System Folgendes aufweist:
(a) eine vierpolige, kreisförmige, zentral geladene, Energie-ausgeglichene magnetische Vorrichtung in einer Vier-Magnete-Anordnung wechselnder Polarität, in welcher die magnetischen Pole lediglich über eine Distanz voneinander getrennt sind, die einen magnetischen Einflussbereich auf alle benachbarten Pole ermöglicht, um das Zünden von Aktionspotenzialen sensorischer Neuronen von Säugetiere zu modulieren oder zu unterdrücken;
(b) einen den Fluss fokussierenden Ring, der die Vier-Magnete-Anordnung auf ihrem äußeren Umfang umgibt, und der im Wesentlichen in der Mitte zwischen dem oberen und dem unteren Ende der magnetischen Pole positioniert ist; und
(c) ein Fluss-Rücklauf-Ring, entfernt von der Membranoberfläche, zur Rückholung des magnetischen Flusses, wodurch die Stärke und der Gradient verändert wird, ohne die zentrale Ladungssymmetrie und -homogenität des dreidimensionalen wechselnden Gradientenfeldes zu verändern, das mit der Membran in Kontakt ist;
**dadurch gekennzeichnet, dass**
(d) das System einen Epiduralkatheter mit einschließt; und
(e) die magnetische Vorrichtung dazu geeignet ist, in den Epiduralkatheter eingesetzt zu werden.

2. System nach Anspruch 1, wobei das System eine statische magnetische Vorrichtung zur Produktion eines magnetischen Feldes zur Behandlung verschiedener Krankheiten bereitstellt.

3. System nach Anspruch 2, wobei das System einen statischen magnetischen Pol ähnlicher Polarität auf der äußeren Oberfläche des den Fluss fokussierenden Ringes bereitstellt, und zwar benachbart zu der geometrischen Seite der Pole der Magnete, derart, dass der statische magnetische Pol auf die geometrische Seite der Pole der Magnete mit einem Winkel von 45 bis 90 Grad ausgerichtet ist.

4. System nach einem der vorstehenden Ansprüche, mit einer wechselnden, vierpoligen Anordnung, in welcher jeder der Pole in Form von zwei Kegeln vorliegt, die an der Mantellinie des Kegels mit der Spitze der zwei Kegel verbunden sind, die in einer senkrechten Achse der zirkulären Mantellinie liegen.

5. System nach einem der vorstehenden Ansprüche, bei welchem die Magnete derart ausgebildet sind, dass sie einen dreidimensionalen wechselnden Gradienten von mindestens 0,25 mT/mm bilden.

6. System nach einem der vorstehenden Ansprüche, mit einer Vielzahl an magnetischen Vorrichtungen, die in einer helikalen Konfiguration entlang dem Katheter angeordnet sind.

## Revendications

1. Système pour modifier la distribution de charges sur des membranes vivantes avec une stabilisation de fonction de l'intégrité électrique physique des membranes, le système comprenant :
(a) un dispositif magnétique équilibré en énergie, à charge centrée circulaire et quadripolaire, dans une matrice à quatre aimants de polarité alterné, dans lequel les pôles magnétiques sont séparés seulement par une distance qui permet à une sphère magnétique d'influence sur tous les pôles adjacents de moduler ou supprimer le déclenchement de potentiel d'action de neurones sensoriels de mammifère ;
(b) un anneau de focalisation de flux entourant la matrice à quatre aimants sur son périmètre extérieur disposé sensiblement à mi-chemin entre le dessus et la base des pôles magnétiques ;et
(c) un anneau de retour de flux éloigné de la surface de membrane pour retourner le flux magnétique, altérant ainsi la force et le gradient sans altérer la symétrie de charge de centre et son homogénéité dans le champ de gradient profond à trois dimensions qui est en contact avec la membrane ;
**Caractérisé en ce que**
(d) le système inclut un cathéter épidural ;
(e) le dispositif magnétique est adapté à être placé dans ledit cathéter épidural.

2. Système selon la revendication 1, **caractérisé en qu'**il comporte un dispositif magnétique statique pour la production d'un champ magnétique en vue du traitement de maladies variées.

3. Système selon la revendication 2, **caractérisé en qu'**il comporte un pôle magnétique statique de polarité identique sur la surface extérieure de l'anneau de focalisation de flux, adjacent aux côtés géométriques des pôles des aimants, de façon que le pôle magnétique statique soit orienté vers les côtés géométriques des pôles des aimants, selon un angle de 45 à 90°.

4. Système selon l'une des revendications précédentes, **caractérisé en qu'**il comprend une matrice quadripolaire alternée, dans laquelle chacun des pôles est sous la forme de deux cônes joints selon leur directrice avec le sommet des deux cônes disposés dans un axe perpendiculaire de la directrice circulaire.

5. Système selon l'une des revendications précédentes, **caractérisé en ce que** les aimants sont configurés pour engendrer un gradient profond, à trois dimensions, d'au moins 0,5mT/mm.

6. Système selon l'une des revendications précédentes, **caractérisé en ce qu'**il comprend des dispositifs magnétiques disposés selon une configuration en hélice le long du cathéter.
